# EUROPEAN PATENT SPECIFICATION

(11) **EP 0 965 340 B1**
(45) Date of publication and mention of the grant of the patent: **13.09.2006**
(21) Application number: 97949140.4
(22) Date of filing: 18.12.1997
(51) Int. Cl.: A61K 31/23, A61K 9/06, A61K 31/232

(54) **DERMATOLOGIC PREPARATION**
DERMATOLOGISCHE ZUSAMMENSETZUNGEN
PREPARATION DERMATOLOGIQUE

(30) Priority: 23.12.1996 JP 35520596
(43) Date of publication of application: 22.12.1999
(73) Proprietor: NIPPON SUISAN KAISHA, LTD., Tokyo 100-0004 (JP)
(72) Inventor: IMAI, Yukie, Nippon Suisan Kaisha, Ltd., Hachioji-shi Tokyo 192 (JP); DOISAKI, Nobushige, Nippon Suisan Kaisha, Ltd., Hachioji-shi Tokyo 192 (JP); SHIMIZU, Nobuyoshi, Nippon Suisan Kaisha, Ltd., Hachioji-shi Tokyo 192 (JP)
(74) Representative: TBK-Patent
(86) International application number: PCT/JP1997/004680
(87) International publication number: WO 1998/027978

(56) References cited:
- JP-A- 7 126 160
- JP-A- 8 109 128
- JP-T- 4 500 971
- JP-T- 4 500 972
- JP-T- 4 500 973
- JP-T- 4 500 974
- JP-T- 4 501 113
- JP-T- 4 501 114
- JP-T- 4 501 115
- JP-T- 4 501 116
- SHIMADA, YUJI ET AL: "Production of structured lipid containing docosahexaenoic and caprylic acids using immobilized Rhizopus delemar lipase" JOURNAL OF FERMENTATION AND BIOENGINEERING , 81(4), 299-303 CODEN: JFBIEX; ISSN: 0922-338X, 1996, XP002278286
- DATABASE WPI Section Ch, Week 198905 Derwent Publications Ltd., London, GB; Class D23, AN 1989-037306 XP002278287 & JP 63 312398 A (NISSHIN OIL MILLS LTD) 20 December 1988 (1988-12-20)

## Description

### Field of the Invention

This invention relates to a dermatologic preparation, in particular the one which is reduced in side effects and has excellent percutaneous absorbability, and contains an unsaturated fatty acid.

### Background of the Invention

For treatment of allergic diseases such as psoriasis and atopic dermatitis, administration of antiallergic drugs or steroids, and dietetic therapy to avoid contact with allergens have long been employed. Among these, administration of steroids has been the leading therapy, though because of severe adverse effects of steroidal drugs one should be always careful in balancing between the efficacy and the adverse reactions and avoid repeated administration. Meanwhile, some unsaturated fatty acids such as eicosapentaenoic acid and docosahexaenoic acid have been found to have therapeutic effects on allergic diseases, and thus these acids are expected to be useful as anti-inflammatory agents with few adverse effects. The anti-allergic effects are assumed to consist mainly in the inhibition of synthesis of prostaglandins and leukotrienes via the antagonism against arachidonic acid.

It has long been known that deficiency of arachidonic acid causes symptoms such as dry skin and keratinization. For treatment of these symptoms, supplement of linoleic acid or arachidonic acid is effective. Patients with atopic dermatitis have abnormalities in composition of fatty acids constituting ceramide, the intercellular lipid among corneocytes, such as decreased linoleic acid level and increased oleic acid level (Clinical Dermatology, 46, p117-120, 1992). In addition, it has been suggested that sebum-constituting fatty acids may be substituted with ceramide-constituting fatty acids (J. Invest Dermatol, 87, p733, 1986), and thus endermic supply of linoleic acid is expected to be effective in treatment of atopy.

These fatty acids have conventionally been used as dermatologic preparations in the form of glycerides, fatty acids as such or the salts thereof, lower-alkyl esters, or phospholipids. However the percutaneous absorbability of these derivatives is so poor that they were rarely effective. Moreover they had disadvantages in use, as they were sticky, stained clothes, and were liable to be oxidized to emit unpleasant odor. Phospholipids were difficult to be purified, so that preparations of high purity were hardly available.

WO 90/04 011 A discloses the triglycerides 2-arachidoyl-1,3-dioctanoyl glycerol and 1,3-didecanoyl glycerol, while the use of these triglycerides for dermatological preparations is contemplated.

WO 90/04 009 A is directed to the triglyceride 2-"soy" -1,3-didecanoyl glycerol, wherein the term "soy" denotes saturated and unsaturated fatty acids having 16 or 18 carbon atoms. Furthermore, the possibility of the use of these triglycerides for dermatological preparations is described.

WO 90/04 010 A is directed to the triglyceride 2-eicosapentaenoyl-1,3-didecanoyl glycerol, the use of this triglyceride for the preparation dermatological formulations is discussed.

### Disclosure of the Invention

As a result of researches of the inventors to achieve the purpose, it was found that a long-chain unsaturated fatty acid, when forms a triglyceride together with a medium-chain fatty acid in the same molecule, has excellent percutaneous absorbability. Surprisingly such a triglyceride showed an improved stability against oxidation and was less liable to emit unpleasant odor. The inventors confirmed that the triglyceride, when applied to the affected part of the skin, showed excellent absorbability without emitting unpleasant odor, and re-confirmed the therapeutic effect known about long-chain unsaturated fatty acids in allergic diseases, including alleviation of itch, reduction of inflammatory area, etc. Thus the inventors have accomplished the present invention.

Namely, the gist of this invention is a dermatologic preparation characterized in that the preparation contains as the active ingredient a triglyceride in which a medium-chain fatty acid residue and a long-chain unsaturated fatty acid residue are contained together in a molecule.

This invention is a dermatologic preparation characterized in that it contains a triglyceride represented by the general formula (1) 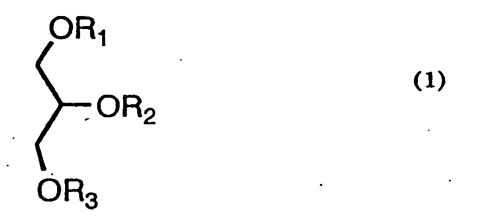 wherein R₁, R₂, and R₃ are defined according to claim 1.

The above-mentioned triglyceride is prepared from a long-chain unsaturated fatty acid and/or a derivative thereof as the starting material, each having a purity of preferably 90% or higher, more preferably 95% or higher.

### Best Embodiments of the Invention

In this invention, the medium-chain fatty acids are fatty acids having 6 to 12 carbon atoms in a molecule, being exemplified by capric acid (decanoic acid), caprylic acid (octanoic acid), and caproic acid (hexanoic acid). The long-chain unsaturated fatty acids in this invention are eicosapentaenoic acid and docosahexaenoic acid.

The dermatologic preparations of this invention are usable for treatment of various diseases resulting from allergic diseases such as psoriasis and atopic dermatitis, that is, they are usable as drugs for therapy, relief (alleviation of symptoms), maintenance (prevention of aggravation), or prevention of the diseases. Such diseases are exemplified by psoriasis and atopic dermatitis, and this invention applies to all diseases with dermatologic symptoms such as dry skin and keratonization which manifest owing to inhibited synthesis of prostaglandins and leukotrienes as a result of antagonism against arachidonic acid, or owing to deficiency of arachidonic acid.

The dermatologic preparation of this invention may be the triglyceride, the active ingredient, which can be applied as it is to the skin as a dermatologic preparation, or in any other dosage form as far as it is able to deliver the active ingredient directly to the skin, for example, ointments, emulsions, gels, cataplasms, tapes, lotions, patches, aerosols, etc.

To the dermatologic preparation of this invention, may be added as needed an additive known in the field of preparations such as a base for external preparations, an absorption-facilitator, an antioxidant, an emulsifier, a humectant, an antiseptic, etc., and/or another drug, such as an anti-inflammatory drug, anti-allergic drug, etc.

The above-mentioned form applicable directly to the skin can be obtained by addition of white soft paraffin, yellow bees wax, liquid paraffin, polyethyleneglycol, etc. to the active ingredient triglyceride, followed by warming, if necessary, and kneading. Cataplasms and tapes can be obtained by mixing and kneading of the active ingredient triglyceride with a tackifier such as rosin and acrylic acid alkyl ester polymer, followed by spreading on a sheet of non-woven fabric. Inhalants can be obtained by dissolving or dispersing the active ingredient triglyceride in an aerosol such as pharmaceutically acceptable inert gas (nitrogen gas, carbon dioxide gas, etc.), followed by filling in a pressure-resistant container.

The content of the active ingredient in the dermatologic preparation of this invention is not specifically limited. The dose is determined appropriately according to the disease to be treated, age, body weight, and conditions of the patient, the symptoms, the dosage form of the preparation, etc.

The triglyceride used in this invention can be produced by the known methods. Namely, any method may be used as far as it can produce the aimed compound, such as random interesterification in the presence of an alkali catalyst, interesterification using an enzyme or acidolysis, or condensation between glycerol and a fatty acid. The triglyceride can be purified by a well-known method in the field of fat and oil, such as degumming, neutralization, bleaching, etc.

The long-chain unsaturated fatty acid and/or a derivative thereof used as a starting material in this invention is a fatty acid or an ester thereof, glyceride, salt, acid chloride, acid anhydride, and the like, i.e. a compound having a residue of a long-chain unsaturated fatty acid. As the starting material of the long-chain unsaturated fatty acid in the triglyceride of this invention, natural fat and oil or a hydrolyzate thereof may be used as it is, or a concentrate of an unsaturated fatty acid by urea addition or by other procedures, or a highly purified product obtained by conversion into a lower alkyl ester followed by distillation, etc. may also be used. However the procedures are not limited only to those described above.

The active ingredient triglyceride in the dermatologic preparation of this invention was shown to have no toxicity as described in the Examples below. Some of such triglycerides have already been used as enteral nutrients and had little or no cytotoxicity when given at the pharmacologically effective dose.

The dermatologic preparation of this invention is explained in more concrete in the following examples, though this invention is not limited at all by these examples.

### Reference Example 1

### Production of a dermatologic prepration for comparison

Refined fish oil (containing 28% of EPA) and a medium-chain fatty acid triglyceride were mixed in the ratio of about 1 to 2 (molar ratio) and subjected to random interesterification in the presence of an alkali catalyst. The resulting triglyceride was purified by a conventional method, and δ-tocopherol was added to the concentration of 0.5% (by weight) as an antioxidant.

The stability of the dermatologic preparation related to this invention was tested.

Twenty gram of the above-mentioned oil was placed in a 30-ml brown bottle and stored at 25°C in a dark place with the bottle open. The peroxide value was determined on days 0, 6, and 10 by the thiocyanate method.

The result is shown in Table 1.

### Reference Example 2

### Production of a dermatologic preparation for comparison

The refined fish oil, the medium-chain fatty acid triglyceride, and the antioxidant used in the Reference Example 1 were mixed mechanically at the same ratio as in the Reference Example 1.

The stability of this dermatologic preparation was tested for comparison.

As did in the Reference Example 1, 20 g of the above-mentioned oil was placed in a 30-ml brown bottle and stored at 25°C in a dark place with the bottle open. The peroxide value was determined on days 0, 6, and 10 by the thiocyanate method.

The result is shown in Table 1.

**Table 1**

| | peroxide value | | |
|---|---|---|---|
| days | 0 | 6 | 10 |
| Reference Example 1 | 0.20 | 0.71 | 1.22 |
| Reference Example 2 | 0.78 | 12.84 | 22.74 |

### Reference Example 3

### Production of a dermatologic preparation for comparison

1.0 g of 1,3-dioctanoin and 1.1 g of eicosapentaenoic acid were dissolved in 10 ml of dichloromethane, to which 360 mg of dimethylaminopyridine and 0.6 g of dicyclohexylcarbodiimide were added, and the mixture was stirred at -5°C for 3 hours. The reaction mixture was filtered, the aimed product was extracted from the mother liquor, and purified by the silica gel column chromatography, to give 1,3-dioctanoyl-2-eicosapentaenoyl glycerol. To this was added δ-tocopherol as an antioxidant to the concentration of 0.5% (by weight).

Feeling on use of the dermatologic preparation related to this invention was evaluated.

To 900 µl of the mixture of glycerol, ethanol, and water in the ratio of 1:2:2 (by volume), was added 100 µl of the above-mentioned oil, and the resultant mixture was applied and spread onto the volar cubital region. The moist feeling after 3 minutes was evaluated.

Also the intensity of unpleasant odor at the region was evaluated 1 hour after application.

The result is shown in Table 2.

### Example 1

### Production of a dermatologic preparation related to this invention

Except that "1,2-dioctanoin" was used in place of "1,3-dioctanoin" in the Reference Example 3, the production and the test of the product were performed in the same manner as described in Reference Example 3.

The resultant dermatologic preparation was also examined for feeling on use in the same manner as described in Reference Example 3.

The result is shown in Table 2.

### Reference Example 4

### Production of a dermatologic preparation for comparison

Ethyl eicosapentaenoate and trioctanoin were mixed in the ratio of 1:2 (molar ratio). To the mixture was added δ-tocopherol as an antioxidant to the concentration of 0.5% (by weight).

Also this preparation for comparison was tested for feeling on use in the same manner as in Reference Example 3.

The result is shown in Table 2.

### Reference Example 5

### Production of a dermatologic preparation for comparison

Eicosapentaenoic acid and trioctanoin were mixed in the ratio of 1:2 (molar ratio). To the mixture was added δ-tocopherol as an antioxidant to the concentration of 0.5% (by weight).

Also this preparation for comparison was tested for feeling on use in the same manner as in Reference Example 3.

The result is shown in Table 2.

### Reference Example 6

### Production of a dermatologic preparation for comparison

Sodium eicosapentaenoate and trioctanoin were mixed in the ratio of 1:2 (molar ratio) to give a suspension. To the suspension was added δ-tocopherol as an antioxidant to the concentration of 0.5% (by weight).

Also this preparation for comparison was tested for feeling on use in the same manner as in Reference Example 3.

The result is shown in Table 2

**Table 2**

| **Moist feeling** | **dry** | **moist** | **tacky** | **oily** |
|---|---|---|---|---|
| Reference Example 1 | 0 subject | 4 subjects | 1 subject | 0 subject |
| Reference Example 2 | 0 | 0 | 1 | 4 |
| Reference Example 3 | 0 | 5 | 0 | 0 |
| Example 1 | 0 | 4 | 1 | 0 |
| Reference Example 4 | 0 | 0 | 0 | 5 |
| Reference Example 5 | 0 | 0 | 2 | 3 |
| Reference Example 6 | 0 | 1 | 4 | 0 |
| **Unpleasant odor** | **strong** | **weak** | **slight** | **none** |
| Reference Example 1 | 0 | 0 | 1 | 4 |
| Reference Example 2 | 0 | 1 | 3 | 1 |
| Reference Example 3 | 0 | 0 | 0 | 5 |
| Example 1 | 0 | 0 | 1 | 4 |
| Reference Example 4 | 0 | 2 | 3 | 0 |
| Reference Example 5 | 0 | 1 | 4 | 0 |
| Reference Example 4 | 0 | 0 | 5 | 0 |

### Example 4

The dermatologic preparations of Reference Example 1 and the dermatologic preparations for comparison of Reference Examples 1 to 5 were evaluated for itch and the area of inflammation 1 week after application.

To the affected regions of 3 atopic patients (1 male adult, 1 female adult, and 1 child), was applied an adequate amount of each oil produced in Example 1 and Reference Examples 1 to 5 after bathing every day. After application for 1 week, itch and the inflammatory area were evaluated. During the treatment period, the drugs used previously were continued. In the child who could not describe itch, itch was evaluated based on the behavior of scratching at the affected region.

The result is shown in Table 3.

**Table 3**

| | itch | | | inflammatory area | | |
|---|---|---|---|---|---|---|
| | improved | no change | worsened | reduced | no change | widened |
| Reference Example 1 | 3 patients | 0 patient | 0 patient | 2 patients | 1 patient | 0 patient |
| Reference Example 2 | 1 | 2 | 0 | 1 | 2 | 0 |
| Reference Example 3 | 3 | 0 | 0 | 3 | 0 | 0 |
| Example 1 | 3 | 0 | 0 | 2 | 1 | 0 |
| Reference Example 4 | 1 | 2 | 0 | 0 | 0 | 0 |
| Reference Example 5 | 0 | 3 | 0 | 0 | 3 | 0 |

### Reference Example 7

### Formulation into cream

Cream was prepared by combining the ingredients listed in Table 4.

First, propyleneglycol was added to purified water, and the mixture was heated to 70°C and used as the aqueous phase. Separately, oily ingredients were mixed and melted by heating to 70°C and stirring, which was used as the oily phase. The oily phase was added to the aqueous phase, and cooled by stirring to give cream.

The cream thus prepared was stored at 25°C in a dark place with the container open, and a sensory test was performed for evaluation of unpleasant odor emitted.

The result is shown in Table 5.

**Table 4**

| | | |
|---|---|---|
| aqueous ingredients | propyleneglycol | 5.0% |
| | purified water | 64 % |
| oily ingredients | oil of Reference Example 1 | 3.0% |
| | stearic acid | 2.0% |
| | cetanol | 7.0% |
| | reduced lanolin | 2.0% |
| | squarane | 4.0% |
| | octyldodecanol | 6.0% |
| | poly(oxyethylene) cetyl ether | 3.0% |
| | glycerol monostearate | 2.0% |
| | perfume | 0.3% |
| | antioxidant | 0.5% |

### Reference Example 8

### Formulation into cream

Except that the "oil of Reference Example 2" was used in place of the "oil of Reference Example 1", cream was prepared with the same combination of ingredients and by the same procedure as in Reference Example 7.

The resultant cream was stored at 25°C in a dark place in an open container and a sensory test was performed for evaluation of unpleasant odor emitted, in the same manner as in Reference Example 7.

The result is shown in Table 5.

**Table 5**

| Cream storage test | | | | |
|---|---|---|---|---|
| days | 0 | 1 | 3 | 7 |
| Reference Example 7 | ○ | ○ | ○ | ○ |
| Reference Example 8 | ○ | ○ | ○ | Δ |

| | | | | |
|---|---|---|---|---|
| ○: no unpleasant odor, Δ: slight unpleasant odor, ×: unpleasant odor | | | | |

### Industrial Applicability

As shown in the above-mentioned Examples, the dermatologic preparation of this invention is extremely stable against oxidation, and excellent in percutaneous absorbability with decreased generation of unpleasant odor. Moreover, it was confirmed that the preparation maintains the therapeutic effects known long about the long-chain unsaturated fatty acid against allergic diseases, such as alleviation of itch, reduction of the inflammatory area, etc. Therefore the dermatologic preparation of this invention is expected to be an excellent therapeutic for treatment of allergic diseases such as atopic dermatitis and psoriasis.

## Claims

1. A dermatologic preparation **characterized in that** it contains a triglyceride represented by the general formula (1): 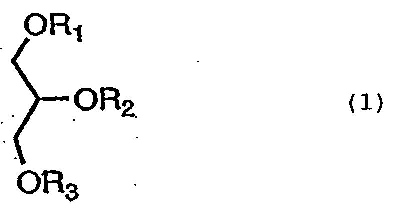 wherein R₁ represents eicosapentanenoic acid or docosahexaenoic acid and the remaining residues R₂ and R₃ each are of a medium-chain fatty acid, as the active ingredient.

2. A dermatologic preparation as claimed in claim 1, wherein the triglyceride is prepared from eicosapentanenoic acid or docosahexaenoic acid of purity of 90 % or higher or/and a derivative thereof as the starting material.

3. A dermatologic preparation as claimed in claim 1, wherein the triglyceride is prepared from eicosapentanenoic acid or docosahexaenoic acid of purity of 95 % or higher or/and a derivative thereof as the starting material.

## Patentansprüche

1. Dermatologische Zusammensetzung, **dadurch gekennzeichnet, dass** sie ein Triglycerid als aktiven Inhaltsstoff enthält, das durch die allgemeine Formel (1) dargestellt wird, 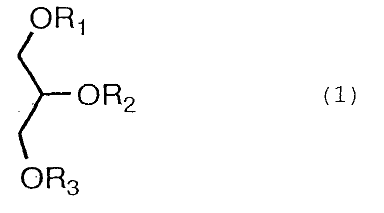 wobei R₁ Eicosapentaensäure oder Docosahexaensäure darstellt und die verbleibenden Reste R₂ und R₃ jeweils mittelkettige Fettsäuren sind.

2. Dermatologische Zusammensetzung nach Anspruch 1, wobei das Triglycerid aus Eicosapentaensäure oder Docosahexaensäure mit einer Reinheit von 90% oder höher, und/oder einem Derivat davon, als Ausgangsmaterial hergestellt wird.

3. Dermatologische Zusammensetzung nach Anspruch 1, wobei das Triglycerid aus Eicosapentaensäure oder Docosahexaensäure mit einer Reinheit von 95% oder höher, und/oder einem Derivat davon, als Ausgangsmaterial hergestellt wird.

## Revendications

1. Préparation dermatologique **caractérisée en ce qu'**elle contient un triglycéride représenté par la formule générale (1) : 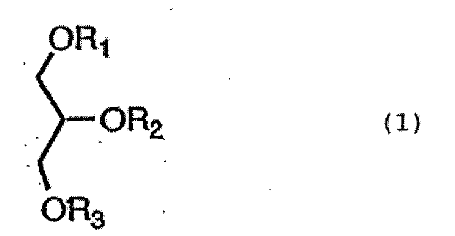 dans laquelle R₁ représente un acide eicosapentanénoïque ou acide docosahexaénoique et les résidus restants R₂ et R₃ sont chacun un acide gras à chaîne moyenne, en tant qu'ingrédient actif.

2. Préparation dermatologique telle que revendiquée dans la revendication 1, dans laquelle le triglycéride est préparé à partir d'un acide eicosapentanénoïque ou acide docosahexaénoïque ayant une pureté de 90 % ou plus élevée ou/et d'un dérivé de celui-ci en tant que produit de départ.

3. Préparation dermatologique telle que revendiquée dans la revendication 1, dans laquelle le triglycéride est préparé à partir d'un acide eicosapentanénoïque ou acide docosahexaénoïque ayant une pureté de 95 % ou plus élevée ou/et d'un dérivé de celui-ci en tant que produit de départ.
